(19) Europäisches Patentamt
European Patent Office
Office européen des brevets

(11) Publication number: **0 137 444**
**B1**

(12) **EUROPEAN PATENT SPECIFICATION**

(45) Date of publication of patent specification: **31.01.90**

(51) Int. Cl.$^5$: **C 07 D 309/30**

(21) Application number: **84111759.1**

(22) Date of filing: **02.10.84**

(54) **Processes for preparing 6(R)-[2-[8(S)(2,2-dimethylbutyryloxy)-2(S),6(S)-dimethyl-1,2,3,4,4a(S),5,6,7,8,8a(S)-Decahydronaphthyl-1(S)]ethyl]-4(R)-hydroxy-3,4,5,6-tetrahydro-23H-pyran-2-one.**

(30) Priority: **11.10.83 US 540958**

(43) Date of publication of application:
**17.04.85 Bulletin 85/16**

(45) Publication of the grant of the patent:
**31.01.90 Bulletin 90/05**

(84) Designated Contracting States:
**CH DE FR GB IT LI NL**

(56) References cited:
**EP-A-0 033 537**
**EP-A-0 033 538**
**EP-A-0 094 443**
**GB-A- 885 044**

(73) Proprietor: **MERCK & CO. INC.**
**126, East Lincoln Avenue P.O. Box 2000**
**Rahway New Jersey 07065-0900 (US)**

(72) Inventor: **Sletzinger, Meyer**
**135 Rockview Avenue**
**North Plainfield New Jersey 07060 (US)**
Inventor: **Verhoeven, Thomas R.**
**106 Oak Lane**
**Cranford New Jersey 07016 (US)**
Inventor: **Volante, Ralph P.**
**22 Hawthorne Lane**
**East Windsor New Jersey 08520 (US)**

(74) Representative: **Abitz, Walter, Dr.-Ing. et al**
**Abitz, Morf, Gritschneder, Freiherr von**
**Wittgenstein Postfach 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 137 444 B1**

**Description**

This invention is concerned with novel processes for the preparation of the compound of structural formula:

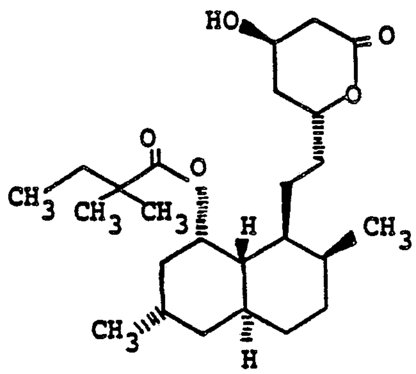

I

which comprises the steps in either order of (1) reduction of the two double bonds of mevinolin; and (2) methylation of the 2-methylbutyryloxy group of mevinolin to form the 2,2-dimethylbutyryloxy group.

Compound I is an antihypercholesterolemic agent useful in the treatment of atherosclerosis, hyperlipemia, familial hypercholesterolemia and like disorders.

Mevinolin, the starting material for the novel process of this invention is a fermentation product described in U.S. Patent 4,231,938.

Hydrogenation products, including tetrahydromevinolin are described in U.S. Patent 4,351,844.

Compound I and processes for its preparation are disclosed in published European applications EP—A—0033538 and EP—A—0094443, the latter being filed before but published after the priority date of the subject application. However, the processes disclosed involve five distinct chemical steps from mevinolin involving 1) reduction; 2) de-esterification of the 8-ester group; 3) protection of the 4-hydroxy of the pyranone group; 4) re-esterification with the desired 2,2-dimethylbutyryl; and 5) deprotection of the 4-hydroxy, not necessarily in that order.

Now, with the present invention there are provided novel processes for the preparation of Compound I involving only two chemical steps resulting in overall yields much higher than those realized by the prior art, with the expenditure of much less time, labor and materials.

The novel processes of this invention are as depicted below:

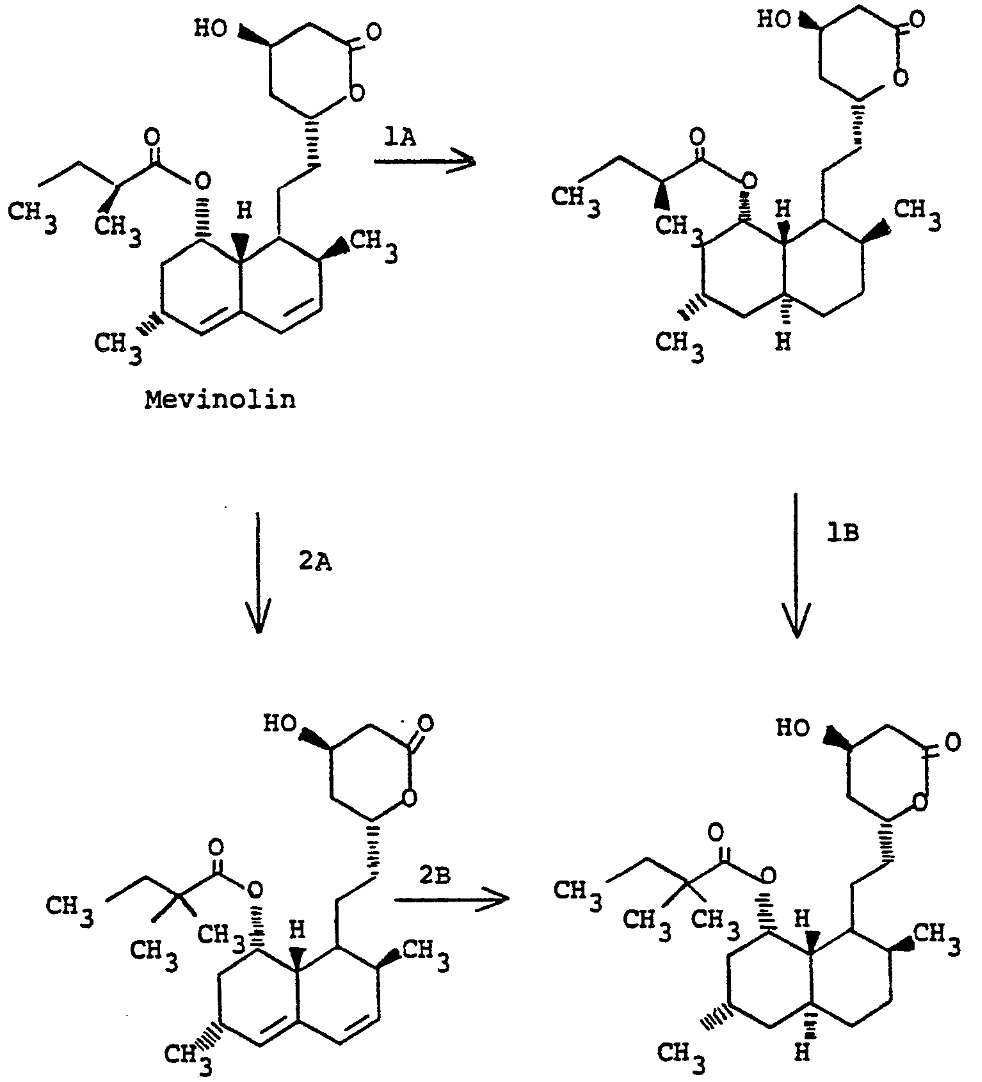


Mevinolin

The reduction of the diene system of mevinolin described in the prior art, U.S. Patent 4,351,844 utilized a $PtO_2$ catalyst, in ethyl acetate at room temperature and atmospheric pressure for about one hour.

However, using those conditions, at best a 60:40 mixture of *trans*:*cis* tetrahydromevinolin was produced. Furthermore, the desired *trans* compound could be isolated from this mixture only after extensive silica-gel column chromatographic purification. Attempts to improve this ratio were investigated using a variety of catalysts and solvents and lead to the novel process of the present invention.

The reduction depicted as process steps 1A and 2B comprises treatment of the starting material in an organic solvent with hydrogen in the presence of a platinum on alumina ($Pt/Al_2O_3$) catalyst with agitation.

The preferred catalyst is 5% $Pt/Al_2O_3$ but other catalysts such as 2 to 10% $Pt/Al_2O_3$ are useful.

The preferred solvent is ethyl acetate, but other solvents which can be employed include isopropyl acetate, hexanes, and methylene chloride or mixtures thereof.

The ratio of starting material to catalyst, by weight is about 3/1 to 6/1 and preferably about 4/1 to 5/1.

The concentration of starting material is about 1 g/30 ml to about 1 g/70 ml and preferably about 1 g/50 ml of solvent.

The reaction proceeds at hydrogen pressures at or above atmospheric pressure, preferably at about 2.07 to 3.45 bar (30 to 50 psi) and most preferably at about 2.76 bar (40 psi) for about 4 to 8 hours at about 15 to 40°C, preferably about 20—25°C.

Employing conditions such as described, the reduction product is about 65 to 75% the desired *trans* isomer which can be isolated and separated from any by-products by direct crystallization in about 60% overall yield with a purity greater than 95%.

The process step depicted as 2A or 1B comprises C-methylation at the 2-position of the 2-methylbutyryloxy group at the 8-position of the polyhydronaphthalene moiety of mevinolin or its tetrahydro analog. The lactone compound is first converted to an alkali metal salt, preferably the potassium salt, of the dihydroxycarboxylate. Although any conceivable process for preparing a dry salt would suffice, it is convenient to add a stoichiometric amount of aqueous potassium hydroxide to a solution of the lactone

starting material in a hydrocarbon solvent such as benzene, toluene, or cyclohexane containing a small amount of a $C_1$—$_3$ alkanol, preferably isopropanol, ethanol or methanol, stirring for a few minutes to about an hour and finally concentrating to dryness *in vacuo*. The residue is subjected to rigorous drying such as by azeotropic distillation with cyclohexane or toluene, as the actual methylation procedure that follows proceeds properly only under rigorously anhydrous conditions.

The dry alkali metal salt is dissolved in an ethereal solvent such as tetrahydrofuran, diethyl ether, or 1,2-dimethoxyethane, cooled to about −60 to about −25°C, preferably −35° to −30°C, and treated with an excess of an alkali metal amide, wherein the alkali metal is lithium, sodium or potassium, preferably lithium, and the amides are diethylamide, pyrrolidide, dimethyl amide or diisopropyl amide in an ethereal solvent in a dry inert environment. After about 2 to 8 hours, preferably about 2 hours at about −50 to −25°C, preferably −35° to −30°C, methylbromide, methylchloride or methyl iodide, preferably methyl bromide, or methyl iodide, is added to the mixture while maintaining the low temperature. Treatment with an alkali metal amide and methyl bromide, methyl chloride or methyl iodide as described can be repeated if appreciable quantities of starting material remain. After about 0.5 to about 3 hours, following final addition of methyl bromide, methyl chloride or methyl iodide the reaction mixture is quenched by adding it to an excess of water. To isolate the product the aqueous phase is adjusted to pH 3—6 with a strong mineral acid such as hydrochloric, hydrobromic, sulfuric, or phosphoric acid. The aqueous phase is extracted with cyclohexane or toluene, dried, filtered, refluxed for 3—20 hours and finally concentrated, and filtered.

Recrystallization provides material of greater than 90% purity in about 60—70% yield over the C-methylation step.

Example 1

6(R) - [2 - [8(S)(2,2 - dimethylbutyryloxy) - 2(S),6(S) - dimethyl - 1,2,3,4,4a(S),5,6,7,8,8a(S) - decahydronaphthyl - 1(S)]ethyl] - 4(R) - hydroxy - 3,4,5,6 - tetrahydro - 2H - pyran - 2 - one

*Step A:* Preparation of *trans*-tetrahydromevinolin

750 mg of 5% $Pt/Al_2O_3$ catalyst was suspended in 75 ml of ethyl acetate in a 300 ml autoclave and stirred at 22—25° and 1500 rpm under 2.76 bar (40 psi) of hydrogen pressure for one hour.

A solution of 3 g of mevinolin in 150 ml of ethyl acetate was then added at a rate of 0.5 ml/min. and the reaction temperature was maintained at 20—25°. The final reaction mixture was then stirred under 2.76 bar (40 psi) of hydrogen pressure for an additional 16 hours. The reaction mixture was then filtered through a 5.08—7.62 cm (2—3 inch) plug of filter aid in a 15 ml coarse sintered glass funnel. The filter cake was washed with about 25 ml of ethyl acetate and the combined filtrates were concentrated to about 6 ml. Hexane (54 ml) was added and the mixture was heated to reflux (65—68°). The solution was then cooled to 10° over 2 hours and aged at 10° for 2 hours. The solids were collected by filtration to yield 2.0 g of trans-tetrahydromevinolin (66% yield) of 99+% relative purity.

*Step B:* Preparation of 6(R) - [2 - 8(S)(2,2 - dimethylbutyryloxy) - 2(S),6(S) - dimethyl - 1,2,3,4,4a(S),5,6,7,8,8a(S) - decahydronaphthyl - 1(S)]ethyl] - 4(R) - hydroxy - 3,4,5,6 - tetrahydro - 2H - pyran - 2 - one

A solution of *trans*-tetrahydro mevinolin (5 g, 12.25 mmol) in cyclohexane (100 ml) and isopropanol (12 ml) was prepared under nitrogen. An aqueous solution of potassium hydroxide (4.91 *M*, 2.5 ml, 12.27 mmol) was added in one portion and the two-phase mixture stirred for 0.5 hr. at ambient temperature. The mixture was concentrated via distillation (bath temperature 100°C). The vessel was recharged with 150 ml of cyclohexane and reconcentrated to a volume of 15 ml. The potassium carboxylate solution was diluted with tetrahydrofuran (35 ml) and cooled to −35°C.

A solution of pyrrolidine (3.6 ml, 43.1 mmol) in tetrahydrofuran (30 ml) was cooled to −5°C and a solution of n-butyllithium (27.5 ml, 42.6 mmol, 1.55 *M* in hexane) was gradually added maintaining an internal temperature below 0°C during the addition.

The lithium pyrrolidide thus prepared was added to the cooled solution of the potassium carboxylate via cannula, maintaining an internal temperature below −30°C throughout the addition. The clear yellow solution was aged between −35 to −30°C for 2 hours. A solution of methyl bromide (2.36 g, 24.8 mmol) in tetrahydrofuran was added maintaining an internal temperature of −20°C. The white slurry was aged for 1 hour at this temperature. A solution of pyrrolidine (1.6 ml, 19.16 mmol) in tetrahydrofuran (15 ml) was cooled to −5°C and n-butyllithium (12 ml, 18.6 mmol, 1.55 *M* in hexane) was added maintaining the temperature below 0°C. This solution was gradually added to the reaction mixture maintaining an internal temperature below −30°C. The mixture was aged at −30 to −35°C for 2 hours. A solution of methyl bromide (3.01 g, 31.7 mmol) in tetrahydrofuran was added maintaining an internal temperature of −20°C. The mixture was aged at that temperature for 1 hour.

The mixture was quenched into a vessel containing water (100 ml), the layers separated and the lower (aqueous) phase adjusted to pH 4.5 with 20% aqueous phosphoric acid. The acidified aqueous phase was extracted three times with 100 ml of cyclohexane. The combined cyclohexane extracts were washed twice with 50 ml of water, then dried over sodium sulfate (25 g). The mixture was filtered and slowly concentrated to a volume of 40 ml via distillation over 5 hours. After cooling to ambient temperature, the mixture was filtered to give crude product (4.15—4.25 g) of approximately 90—92% purity. The product was dissolved in methanol (22 gl/g of substrate) and water (6.2 ml/g) with stirring at 65°C while additional water (6.2 ml/g) was added. The mixture was aged at ambient temperature overnight, filtered and dried under vacuum at

50°C to yield pure product (85—92% recovery). Overall yield from *trans*-tetrahydro mevinolin is 67—75%.

*Alternate Step B:*

The potassium salt of *trans*-tetrahydromevinolin (20 g, 49 mmole) is prepared in cyclohexane (400 ml), isopropanol (48 ml) and aqueous potassium hydroxide (10 ml, 4.91 Molar) as described above. The mixture is concentrated by distillation at atmospheric pressure. Additional cyclohexane (450 ml) is added. A total of 600 ml of distillate is collected. A K.F. of less than 70 µg $H_2O$/ml should be observed. The mixture is concentrated to a total volume of 52 ml.

Tetrahydrofuran (280 ml) and pyrrolidine (16 ml) is charged into the vessel and the solution cooled to *less than* −55°C. Butyl lithium (110 ml, 1.55 *M*) is *slowly* added to the well stirred mixture, maintaining an internal temperature *below −55°C* throughout the addition. The mixture is aged at −30 to −35°C for 2.5 hours. Methyl bromide (10.0 g) is bubbled into the solution maintaining an internal temperature of −20 to −25°C. After an age of 15 minutes HPLC analysis is performed to confirm greater than 93% conversion. If less than 93% conversion is observed a second charge of methyl bromide (normally 0.25—0.75 g) is introduced. The mixture is aged at −20 to −25°C for a total of one hour. Tetrahydrofuran (60 ml) and pyrrolidine (6.4 ml) are charged to the reaction mixture and cooled to *less than* −55°C. n-BuLi (48 ml, 1.55 *M*) is slowly added as before maintaining an internal temperature below −55°C during the addition. The mixture is aged for 2 hours at −30 to −35°C. Methyl bromide (12 g) is introduced as described above and the mixture aged for 1 hour at −20 to −25°C. The reaction mixture is quenched into 400 ml of $H_2O$ and worked up and relactonized as described above. The crude yield after filtration of cyclohexane slurry and drying (40°C *in vacuo*) 17.89 g, 86.9% pure. Overall yield is 75.2%.

Example 2

6(R) - [2 - [8(S)(2,2 - dimethylbutyryloxy) - 2(S),6(S) - dimethyl - 1,2,3,4,4a(S),5,6,7,8,8a(S) - decahydronaphthyl - 1(S)]ethyl] - 4(R) - hydroxy - 3,4,5,6 - tetrahydro - 2H - pyran - 2 - one

*Step A:* Preparation of 6(R) - [2 - [8(S)(2,2 - dimethylbutyryloxy) - 2(S),6(S) - dimethyl - 1,2,3,4,4a(S),5,6,7,8,8a(S) - decahydronaphthyl - 1(S)]ethyl] - 4(R) - hydroxy - 3,4,5,6 - tetrahydro - 2H - pyran - 2 - one

Employing the procedure substantially as described in Example 1, Step B, or its alternate but substituting for the tetrahydro-mevinolin used as starting material therein, an equimolar amount of mevinolin, there was produced the title compound (50—64% yield).

*Step B:* Preparation of 6(R) - [2 - [8(S)(2,2 - dimethylbutyryloxy) - 2(S),6(S) - dimethyl - 1,2,3,4,4a(S),5,6,7,8,8a(S) - decahydronaphthyl - 1(S)]ethyl] - 4(R) - hydroxy - 3,4,5,6 - tetrahydro - 2H - pyran - 2 - one

Employing the procedure substantially as described in Example 1, Step A, but substituting for the mevinolin used therein, an equimolar amount of the product from Step A of this Example 2, there was produced the title compound.

**Claims**

1. A process for the preparation of a compound of structural formula:

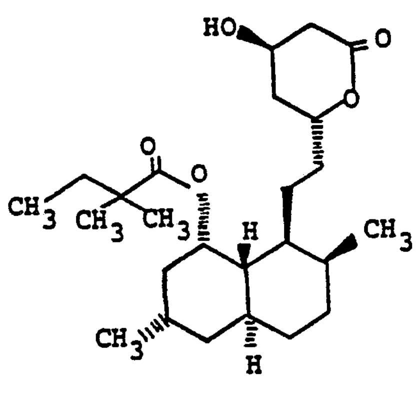

which comprises the steps in either order of

(1) catalytic reduction of the two double bonds of the mevinolin molecule with a platinum on alumina catalyst in an organic solvent with agitation; and

(2) C-methylation of the 2-methylation of the 2-methylbutyryloxy side chain of the mevinolin molecule by conversion of the mevinolin molecule from the lactone form into the alkali metal salt of the dihydroxycarboxylate, methylation with an alkali metal amide and methylating agent selected from methyl chloride, methyl bromide or methyl iodide and adjustment of the pH to 3 to 6 with mineral acid followed by relactonization.

2. The process of Claim 1 wherein the two steps are in the order of reduction followed by C-alkylation.

3. The process of Claim 1 wherein

(1) the reduction step utilizes a 2—10% $Pt/Al_2O_3$ catalyst; the solvent is selected from ethyl acetate, isopropyl acetate, hexanes, and methylene chloride; the hydrogen pressure is atmospheric or higher; and the temperature is 15—40°C.

(2) the C-methylation step is conducted with methyl iodide or methyl bromide in an ethereal solvent at −50 to −25°C in the presence of an alkali metal amide wherein the alkali metal is sodium, potassium or lithium and the amide is diethylamide, dimethylamide, pyrrolidide or diisopropylamide.

4. The process of Claim 3 wherein the two steps are in the order of reduction followed by alkylation.

5. The process of Claim 3 wherein

(1) the reduction step utilizes 5% $Pt/Al_2O_3$, ethyl acetate, 2.76 bar (40 psi) of hydrogen, at 20—25°C; and

(2) the C-methylation step utilizes methyl iodide or methyl bromide, in tetrahydrofuran, at −35° to −30°C in the presence of lithium diethylamide or lithium pyrrolidide.

6. The process of Claim 5 wherein the two steps are in the order of reduction followed by alkylation.

7. A process for the preparation of the compound of structural formula:

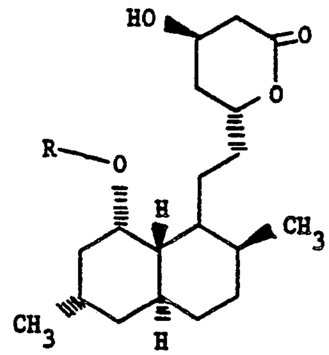

wherein R is 2(S)-methylbutanoyl or 2,2-dimethylbutanoyl which comprises catalytic reduction, with a platinum on alumina catalyst in an organic solvent with agitation, of the compound of structural formula:

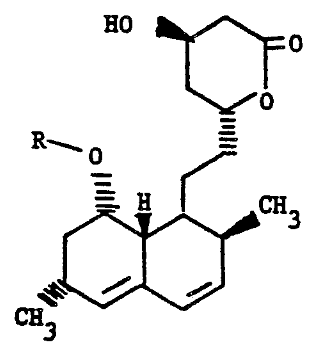

8. The process of Claim 7 wherein the catalyst is 2—10% $Pt/Al_2O_3$; the solvent is selected from ethyl acetate, isopropyl acetate, hexanes, methylene chloride and mixtures thereof, the hydrogen pressure is atmospheric or higher, and the temperature is 15—40°C.

9. The process of Claim 8, wherein the catalyst is 5% $Pt/Al_2O_3$, the solvent is ethyl acetate, the hydrogen pressure is 2.76 bar (40 psi) and the temperature is 20—25°C.

**Patentansprüche**

1. Ein Verfahren für die Herstellung einer Verbindung der Strukturformel:

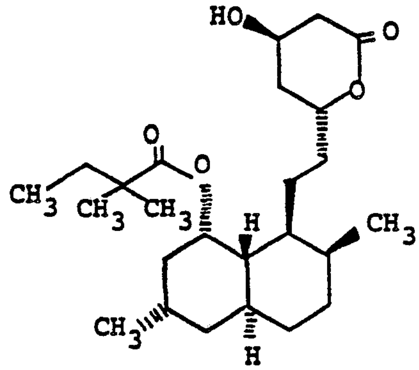

das in beliebiger Reihenfolge die Schritte umfasst der

(1) katalytischen Reduktion der zwei Doppelbindungen des Mevinolin-Moleküls mit einem Platin-auf-Aluminiumoxid-Katalysator in einem organischen Lösungsmittel unter Bewegen; und

(2) C-Methylierung der 2-Methylbutyryloxy-Seitenkette des Mevinolin-Moleküls durch Umwandlung des Mevinolin-Moleküls von der Lactonform in das Alkalimetallsalz des Dihydroxycarboxylats, Methylierung mit einem Alkalimetallamid und einem Methylierungsmittel, ausgewählt aus Methylchlorid, Methylbromid oder Methyljodid und Einstellung des pH's auf 3 bis 6 mit Mineralsäure gefolgt von Relactonisierung.

2. Das Verfahren nach Anspruch 1, worin die zwei Schritte in der Reihenfolge Reduktion gefolgt durch C-Alkylierung sind.

3. Das Verfahren nach Anspruch 1, worin

(1) der Reduktionsschritt einen 2—10% $Pt/Al_2O_3$-Katalysator verwendet; das Lösungsmittel ausgewählt ist aus Ethylacetat, Isopropylacetat, Hexanen und Methylenchlorid; der Wasserstoffdruck Atmosphärendruck oder höher ist und die Temperatur 15—40°C ist;

(2) der C-Methylierungsschritt mit Methyljodid oder Methylbromid in einem etherischen Lösungsmittel bei −50 bis −25°C in Gegenwart eines Alkalimetallamids durchgeführt wird, worin das Alkalimetall Natruim, Kalium oder Lithium ist und das Amid Diethylamid, Dimethylamid, Pyrrolidid oder Diisopropylamid ist.

4. Das Verfahren nach Anspruch 3, worin die zwei Schritte in der Reihenfolge Reduktion gefolgt durch Alkylierung sind.

5. Das Verfahren nach Anspruch 3, worin

(1) der Reduktionsschritt 5% $Pt/Al_2O_3$, Ethylacetat und 2,76 bar (40 psi) Wasserstoff bei 20—25°C verwendet; und

(2) der C-Methylierungsschritt Methyljodid oder Methylbromid in Tetrahydrofuran bei −35° bis −30°C in Gegenwart von Lithiumdiethylamid oder Lithiumpyrrolidid verwendet.

6. Das Verfahren nach Anspruch 5, worin die zwei Schritte in der Reihenfolge Reduktion gefolgt durch Alkylierung sind.

7. Ein Verfahren für die Herstellung der Verbindung der Strukturformel:

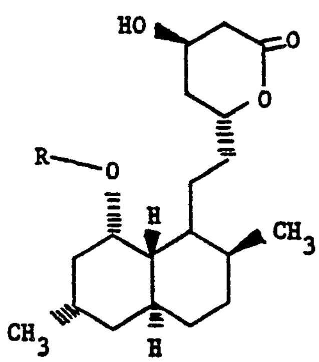

worin R 2(S)-Methylbutanoyl oder 2,2-Dimethyl-butanoyl ist, das die katalytische Reduktion der Verbindung der Strukturformel:

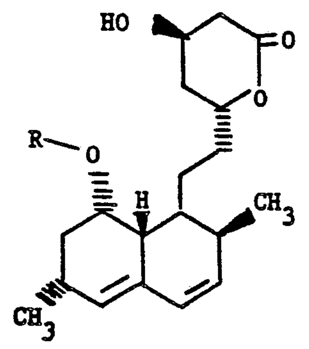

mit einem Platin-auf-Aluminiumoxid-Katalysator in einem organischen Lösungsmittel unter Bewegen umfasst.

8. Das Verfahren nach Anspruch 7, worin der Katalysator 2—10% $Pt/Al_2O_3$ ist; das Lösungsmittel ausgewählt ist aus Ethylacetat, Isopropylacetat, Hexanen, Methylenchlorid und Mischungen davon, der Wasserstoffdruck Atmosphärendruck oder höher ist und die Temperatur 15—40°C ist.

9. Das Verfahren nach Anspruch 8, worin der Katalysator 5% $Pt/Al_2O_3$ ist, das Lösungsmittel Ethylacetat ist, der Wasserstoffdruck 2,76 bar (40 psi) ist und die Temperatur 20—25°C ist.

**Revendications**

1. Procédé de préparation d'un composé de formule structurale:

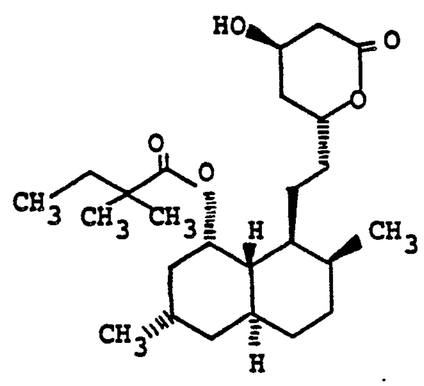

qui comprend les stades suivants dans l'un ou dans l'autre ordre:

(1) réduction catalytique des deux doubles liaisons de la molécule de mevinoline avec un catalyseur d'alumine platinée dans un solvant organique sous agitation; et

(2) C-méthylation de la chaîne latérale 2-méthylbutyryloxy de la molécule de mévinoline par conversion de la molécule de mévinoline à partir de sa forme de lactone en dihydroxycarboxylate de métal alcalin, la méthylation avec un amidure de métal alcalin et un agent de méthylation choisi parmi le chlorure de méthyle, le bromure de méthyle ou l'iodure de méthyle et réglage du pH à une valeur de 3 à 6 avec un acide minéral, qu'on fait suivre d-une relactonisation.

2. Procédé selon la revendication 1, dans lequel l'ordre des deux stades est la réduction suivie de la C-alkylation.

3. Procédé selon la revendication 1, dans lequel

(1) le stade de réduction utilise un catalyseur de 2 à 10% de $Pt/Al_2O_3$; on choisit le solvant parmi l'acétate d'éthyle, l'acétate d'isopropyle, les hexanes et le chlorure de méthylène; la pression d'hydrogène est la pression atmosphérique ou plus élevée; et la température est de 15 à 40°C;

(2) on effectue le stade de C-méthylation avec de l'iodure de méthyle ou bromure de méthyle dans un solvant éthéré à une température comprise entre −50 et −25°C en présence d'un amide de métal alcalin, dans lequel le métal alcalin est le sodium, le potassium ou le lithium et l'amide est le diéthylamide, le diméthylamide, le pyrrolidide ou le diisopropylamide.

4. Procédé selon la revendication 3, dans lequel l'ordre des deux stades est la réduction suivie par l'alkylation.

5. Procédé selon la revendication 3, dans lequel

(1) le stade de réduction utilise 5% de $Pt/Al_2O_3$, l'acétate d'éthyle, une pression de 2,76 bars (40 psi) d'hydrogène à une température de 20 à 25°C; et

(2) le stade de C-méthylation utilise de l'iodure de méthyle ou du bromure de méthyle dans le

tétrahydrofuranne à une température de −35 à −30°C en présence de diéthylamide de lithium ou de pyrrolidide de lithium.

6. Procédé selon la revendication 5, dans lequel l'ordre des deux stades est la réduction suivie par l'alkylation.

7. Procédé de préparation du composé de formule structurale:

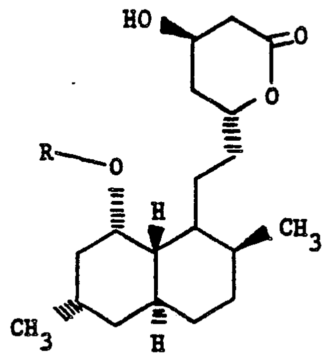

dans laquelle R est un (2)S-méthylbutanoyle ou le 2,2-diméthylbutanoyle, qui comporte une réduction catalytique avec un catalyseur d'alumine platinée dans un solvant organique sous agitation du composé de formule structurale:

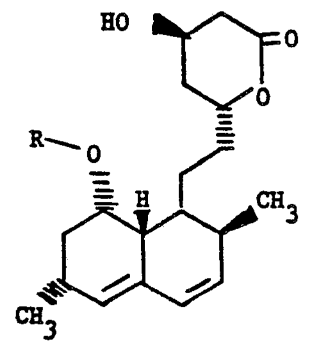

8. Procédé selon la revendication 7, dans lequel le catalyseur est de 2 à 10% $Pt/Al_2O_3$; le solvant est choisi parmi l'acétate d'éthyle, l'acétate d'isopropyle, les hexanes, le chlorure de méthylène et leurs mélanges; la pression d'hydrogène est la pression atmosphérique ou plus élevée et la température est de 15 à 40°C.

9. Procédé selon la revendication 8, dans lequel le catalyseur est de 5% de $Pt/Al_2O_3$, le solvant est l'acétate d'éthyle, la pression d'hydrogène est 2,76 bars (40 psi) et la température est de 20 à 25°C.